# EUROPÄISCHE PATENTSCHRIFT

(11) **EP 3 323 380 B1**
(45) Veröffentlichungstag und Bekanntmachung des Hinweises auf die Patenterteilung: **08.01.2020**
(21) Anmeldenummer: 16199650.9
(22) Anmeldetag: 18.11.2016
(51) Int. Cl.: A61C 17/00, A61C 8/00, A61C 17/02

(54) **BEHANDLUNGSSYSTEM ZUR REINIGUNG EINES MIT BIOFILMVERUNREINIGTEN BAUTEILS, INSBESONDERE EINES IMPLANTAT-TEILS**
TREATMENT SYSTEM FOR CLEANING A COMPONENT WITH BIOFILM IMPURITIES, IN PARTICULAR AN IMPLANT PART
SYSTÈME DE TRAITEMENT DESTINÉ À NETTOYER UN COMPOSANT SOUILLÉ PAR BIOFILM, EN PARTICULIER D'UNE PARTIE D'IMPLANT

(43) Veröffentlichungstag der Anmeldung: 23.05.2018
(73) Patentinhaber: Schlee, Markus, 91301 Forchheim (DE)
(72) Erfinder: Schlee, Markus, 91301 Forchheim (DE)
(74) Vertreter: Tergau & Walkenhorst Patentanwälte PartGmbB

(56) Entgegenhaltungen:
- EP-A1- 2 772 469
- AT-B- 412 002
- US-A1- 2010 038 238
- US-A1- 2016 000 947

## Beschreibung

Die Erfindung betrifft ein Behandlungssystem zur Reinigung eines mit Biofilm verunreinigten Bauteils, insbesondere zur Reinigung von bakteriell verunreinigten Oberflächen von Knochenimplantaten oder Dentalimplantaten, mit zumindest zwei zur Bildung eines elektrischen Stromkreises an eine elektrische Versorgungseinheit angeschlossenen Leiterelementen, von denen eines mit dem behandlungsbedürftigen Bauteil in elektrischen Kontakt bringbar ist.

Ein derartiges Behandlungssystem ist aus der WO2014122188A1 bekannt. Dieses Behandlungssystem ist insbesondere zur Verwendung mit einem Implantat-Teil und zur Reinigung eines Dental-Implantat-Teils vorgesehen. Eine derartige Reinigung eines Implantat-Teils kann wünschenswert oder erforderlich sein, um das Einwachsen oder den Erhalt des zu inserierenden bzw. inserierten Implantats in der Knochensubstanz zu gewährleisten oder zu fördern. An der von Gewebe und Gewebeflüssigkeit umschlossenen festen Oberfläche von Implantaten kann sich nämlich ein Biofilm bilden, der von Bakterien besiedelt wird, die letztlich zu chronischen und wiederkehrenden Infektionen oder auch zu unerwünschtem Knochenabbau führen können. Dieses Erkrankungsbild wird als Periimplantitis bezeichnet. Insbesondere im dentalen Bereich ist ähnlich wie bei der Parodontitis eine Kombination aus vernachlässigter Mundhygiene, Anhaftung von Biofilm an der üblicherweise mikrorauen Oberfläche des Dentalimplantats und weiterer Faktoren ursächlich für das Vollbild der Periimplantitis, welche sich durch eine zunehmende Belastung und Zerstörung des Hart- und Weichgewebes auszeichnet. Die Bereiche, an denen sich das Hart- und/oder Weichgewebe zurückzieht, werden dabei in der Regel mit einem Biofilm überzogen.

Das in der genannten Anmeldung beschriebene Reinigungsverfahren beruht auf dem Konzept, den die Verunreinigung bildenden Biofilm bzw. die Keime ausgehend von der Implantatoberfläche abzutöten und/oder zu entfernen, ohne dabei die Implantatoberfläche zu beschädigen. Dazu ist ein elektrolytischer Prozess vorgesehen, bei dem Ionen (Kationen und/oder Anionen) mittels elektrostatischer Kräfte durch den Biofilm hindurch befördert werden. Diese Ionen reagieren an der Implantatoberfläche chemisch oder elektrochemisch. Durch diese Reaktionen werden neue Stoffverbindungen geschaffen und/oder die Ionen selbst und/oder Teile dieser Ionen in den atomaren Zustand überführt. Darüber hinaus besteht zudem die Möglichkeit, dass die Ionen mit dem Oberflächenmaterial reagieren (z. B. Bildung einer Oxidschicht oder Materialabtrag).

Die keimtötende Wirkung dieses Prozesses basiert auf unterschiedlichen Effekten. Zum einen werden durch das Anlegen einer elektrischen Spannung Ionen aus dem Biofilm selbst (auch aus den Bakterien) zur Anode oder Kathode befördert. Dies kann zur Abtötung von Bakterien und Viren führen. Darüber hinaus können die Ionen, während sie den Biofilm passieren, biochemische Reaktionen eingehen, was ebenfalls zur Abtötung von Bakterien und/oder Viren führen kann. Eine weitere Möglichkeit der Abtötung besteht darin, dass die an der Implantatoberfläche neu gebildeten Stoffverbindungen antibakterielle und/oder antivirale und/oder antifungizide Wirkung besitzen. Dies kann natürlich auch passieren, wenn die Ionen in den atomaren Zustand übergehen.

Das in der genannten Anmeldung beschriebene Behandlungssystem ist für eine besonders einfach gehaltene Handhabung und einen besonders flexiblen Einsatz ausgelegt, ohne dass eine Demontage der Prothetik bei einem nur vergleichsweise leicht von Biofilm befallenen Implantat erforderlich wäre. Dazu umfasst das bekannte System zwei in der Art von Elektroden ausgeführte Leiterelemente, von denen eines direkt in elektrischen Kontakt mit dem behandlungsbedürftigen Bauteil gebracht werden kann, wobei das andere eine Medienkanüle zum Einbringen der ionenhaltigen Flüssigkeit in die Umgebung des behandlungsbedürftigen Bauteils und gleichzeitig durch die Nutzung der elektrischen Leitfähigkeit der Behandlungsflüssigkeit die zur Bildung des geschlossenen Stromkreises notwendigen zweiten Verbindungsleitung zur gemeinsamen elektrischen Versorgungseinheit bildet.

Die konzeptbedingt für den Einsatz des aus der genannten Anmeldung bekannten Behandlungssystems erforderlichen Ionen werden dort bereitgestellt, indem eine geeignete ionenhaltige Behandlungsflüssigkeit bereitgestellt und über die Medienkanüle in den zu behandelnden Raumbereich eingespeist wird. Die Behandlungsflüssigkeit ist dabei zwar bezüglich ihres lonengehalts (beispielsweise bereitgestellt über entsprechende Salze oder Säureanteile) im Hinblick auf die Erfordernisse bezüglich Verträglichkeit, Einwirkung auf das menschliche Gewebe und dergleichen geeignet gewählt und zusammengestellt; andererseits sind aber im Hinblick auf den gewünschten Behandlungserfolg und die Wirksamkeit bei der Abtötung von Keimen gewisse Ionenbestandteile erforderlich, die hinsichtlich der Verträglichkeit und Biokompatibilität nachteilig sein könnten. Zudem kann die zuverlässige Bereitstellung der notwendigen Behandlungsflüssigkeit aufwendig sein.

Der Erfindung liegt daher die Aufgabe zugrunde, ein Behandlungssystem der oben genannten Art anzugeben, mit dem der gewünschte Behandlungserfolg ohne die Notwendigkeit der Zuführung einer ionenhaltigen Behandlungsflüssigkeit ermöglicht wird. Diese Aufgabe wird erfindungsgemäß dadurch gelöst, dass zumindest eines der Leiterelemente im Bereich seines freien Endes als bordotierte Diamantelektrode ausgeführt ist.

Vorteilhafte Ausgestaltungen der Erfindung sind Gegenstand der Unteransprüche. Weitere und/oder alternative vorteilhafte Ausgestaltungen der Erfindung ergeben sich auch aus der Figurenbeschreibung.

Unter "Diamantelektrode" ist dabei insbesondere eine Elektrode zu verstehen, die in ihrem Oberflächenbereich, über den eine Wechselwirkung mit der Umgebung erfolgen kann, aus Diamant als Basismaterial aufgebaut ist, vorteilhafterweise aus bordotiertem Diamant. Insbesondere kann eine solche Diamantelektrode aus einem metallischen oder keramischen Grundkörper aufgebaut sein, der mit einer Diamantbeschichtung versehen ist. Grundsätzlich ist nämlich Diamant für den Einsatz als einzigartiges Elektrodenmaterial geeignet. Dotiert man Diamant mit wenigen Prozent Bor, so entsteht statt dem nicht leitfähigen Diamant ein halbleitender Diamantkristall. Versorgt man diese Diamantkristalle beispielsweise in einer bi-polaren Betriebsweise mit einer Überspannung von wenigen Volt, so zeigt die Diamantoberfläche eine besonders hohe Oxidationskraft, ohne jedoch dabei selbst angegriffen oder gar zerstört zu werden. Bor-dotierter Diamant bietet eine leitfähige Oberfläche zur Produktion von kurzlebigen Hydroxylradikalen oder OH⁻-Ionen. Bei Kontakt mit organischen Verunreinigungen werden diese direkt zu Kohlendioxid (CO₂) und Wasser (H₂O) oxidiert. Überschüssige Hydroxylradikale stabilisieren sich, indem sie mit Wasser und AD-Soda zu Oxidationsmitteln (Aktivsauerstoff) reagieren. Die dabei gebildeten Percarbonate und Peroxide stellen eine Depotwirkung für die Desinfektion bereit, wodurch die Bildung von unerwünschten Mikroorganismen unterbunden wird bzw. eingetragene Keime deaktiviert werden können.

Dementsprechend ist der Einsatz von Diamantelektroden beispielsweise für die elektrochemische Wasseraufbereitung bekannt, wie dies in der WO 2009/052163 A2 beschrieben ist.

Von besonderer Bedeutung für den nunmehr vorgesehenen Einsatzzweck in einem Behandlungssystem der genannten Art ist dabei aber, dass gemeinsam mit und korrespondierend zu den Hydroxylradikalen oder OH⁻ -Ionen infolge der an der Diamantelektrode stattfindenden Zerlegung von Wassermolekülen auch Wasserstoff- oder H⁺ - Ionen entstehen, die für die bevorzugt vorgesehene Behandlung eines mit Biofilm oder anderen angelagerten Verunreinigungen verunreinigten Bauteils besonders bedeutsam sind. Bei geeigneter elektrischer Beschaltung des zu behandelnden Bauteils als Kathode (oder als "Minuspol") des Stromkreises wandern nämlich die an der Diamantelektrode erzeugten H⁺ - Ionen zum Bauteil hin, wobei sie den an dieses angelagerten Biofilm penetrieren. Insbesondere aufgrund der geringen Größe der H⁺ - Ionen können diese den Biofilm bzw. möglicherweise an der Oberfläche angelagerte Kohlenwasserstoffe oder dergleichen vergleichsweise leicht durchdringen und zur Kathode, also zum behandlungsbedürftigen Bauteil, als solches gelangen. An der Kathode, also an der Oberfläche des behandlungsbedürftigen Bauteils, werden die H⁺ - Ionen reduziert und nehmen ein Elektron auf, so dass sich molekularer Wasserstoff (H₂) bildet. Dieser molekulare Wasserstoff entsteht direkt an der Oberfläche des kathodisch geschalteten Bauteils und somit unter dem anhaftenden Biofilm oder den sonstigen anhaftenden Verunreinigungen, wie beispielsweise Kohlenwasserstoffen. Der entstehende Wasserstoff bildet Gasblasen, die anschließend aufsteigen und an die Atmosphäre abgegeben werden. Dabei durchdringen diese Gasblasen den Biofilm bzw. die anderen anhaftenden Verunreinigungen, wobei diese von der Oberfläche des Bauteils abgelöst und "mitgenommen" werden. Durch eine bevorzugt vorgesehene stetige Nachführung einer Spülflüssigkeit, beispielsweise Wasser, werden die abgelösten Verunreinigungen ständig verdünnt und von der Oberfläche des zu behandelnden Bauteils weggeschwemmt, so dass eine zuverlässige und nachhaltige Reinigung erreichbar ist.

Durch den nunmehr vorgesehenen Einsatz einer solchen Diamantelektrode im Behandlungssystem zur Reinigung eines mit Biofilm versehenen Bauteils kann somit erreicht werden, dass die für die konzeptgemäß vorgesehene kombinierte Behandlung aus Beaufschlagung mit elektrischem Strom einerseits und Behandlung mit geeigneten Ionen andererseits notwendigen Ionen nicht unbedingt von extern und über eine aufwendige Versorgung zugeführt werden müssen. Vielmehr können diese Ionen in Form der an der Diamantelektrode entstehenden H⁺ - Ionen während der Prozessführung und durch den Prozess selber vor Ort direkt erzeugt werden. Das solchermaßen modifizierte Behandlungssystem kommt somit ohne nennenswerte Zufuhr aktiver Ionen aus; lediglich die Prozessführung in wässriger Umgebung oder unter Zuführung einer wasserhaltigen oder wasserbasierten Behandlungsflüssigkeit ist notwendig, so dass an der Diamantelektrode die Zersetzung von Wassermolekülen unter Bildung der Hydroxylradikale und der H⁺ - Ionen stattfinden kann.

Die Leiterelemente sind bevorzugt jeweils mit einem Pol einer Stromversorgungseinheit verbunden, so dass durch geeignete Handhabung und Kontaktierung mit entsprechenden leitfähigen Elementen ein elektrischer Strompfad gebildet werden kann. In einer bevorzugten Ausführungsform sind dabei das oder die eigentlichen Leiterelemente in der Art von nadelartig geformten Elektroden ausgebildet, so dass ein Benutzer das jeweilige freie, also nicht über ein Verbindungskabel mit der Stromversorgung verbundene Ende der nadelartigen Elektrode örtlich sehr genau an einem Zielort positionieren und damit den Strompfad sehr genau einstellen kann.

In zusätzlicher oder alternativer vorteilhafter Ausgestaltung ist der als Diamantelektrode ausgestaltete Teil des jeweiligen Leiterelements für eine besonders große effektive Oberfläche ausgestaltet, so dass eine besonders große wirksame Oberfläche für die angestrebte Zerlegung der Wassermoleküle bereitgestellt werden kann. Dazu kann die Diamantelektrode beispielsweise als Hohlzylinder ausgestaltet sein, so dass als effektiv wirksame Fläche sowohl die Außenfläche als auch die Innenfläche des Hohlzylinders zur Verfügung steht. Alternativ oder zusätzlich kann die Oberfläche der Diamantelektrode auch mit einer Struktur, beispielsweise einer Waben- oder Gitterstruktur und/oder mit einer aufgebrachten Rauhigkeit, versehen sein, so dass sich eine entsprechende Vergrößerung der wirksamen Oberfläche selbst bei unveränderten äußeren Abmessungen ergibt. Weiterhin kann bevorzugt die Diamantelektrode vergleichsweise nah am freien Ende des jeweiligen Leiterelements positioniert oder auch als dessen freie Kontaktspitze ausgestaltet sein, so dass die vorgesehene prozessbedingte Produktion der H⁺ - Ionen vergleichsweise nah an dessen vorgesehenem Einsatzort, also zum Beispiel in unmittelbarer Nähe des zu behandelnden Biofilms, stattfinden kann.

Um die Behandlung und die Handhabung zu vereinfachen, können der ionische Strompfad und der Elektronenstrompfad in einer bevorzugten Ausführungsform in einem Bauteil ausgeführt sein. Hierbei können diese nebeneinander oder koaxial ineinander angeordnet sein. Die koaxiale Ausführung kann so gestaltet sein, dass der Elektronenstrompfad innerhalb des lonenstrompfades (oder umgekehrt) ausgeführt sein kann.

Grundsätzlich und konzeptbedingt ist die Funktionsweise des Behandlungssystems nicht von der Zuführung einer gesonderten oder speziellen Behandlungsflüssigkeit abhängig, da die für die Behandlung notwendigen Ionen bei der Prozessführung unmittelbar erzeugt werden. Günstig für die Prozessführung und somit bevorzugt ist aber eine ausreichende und vorteilhafterweise kontinuierliche Zuführung eines Spülmittels, vorzugsweise Wasser oder eine Flüssigkeit auf Wasserbasis, so dass einerseits immer ein ausreichender Wasservorrat in der Umgebung der Diamantelektrode und damit die kontinuierliche Produktion der H⁺ - Ionen gewährleistet ist, wobei andererseits druch den Spülmittelfluss auch die abgelösten Bestandteile des Biofilms oder der Verunreinigungen zuverlässig und kontinuierlich abgeführt werden können.

Alternativ oder zusätzlich ist vorteilhafterweise eine zur Ausbringung einer Behandlungsflüssigkeit vorgesehene Medienkanüle, beispielsweise in Form einer Pipette oder Applikationslanze, vorgesehen. Als Behandlungsflüssigkeit wird dabei bevorzugt eine wasserbasierte oder Wasser enthaltende Flüssigkeit zugeführt, um im Bereich der Diamantelektrode einen zur Erzeugung der Hydroxylradikale und H⁺ - Ionen ausreichenden Wasservorrat bereitzustellen. Vorteilhafterweise ist dabei der Innenraum der Medienkanüle elektrisch leitend mit der elektrischen Versorgungseinheit verbunden, so dass die eingespeiste Behandlungsflüssigkeit selber im Bereich der Medienkanüle den Strompfad zur Versorgungseinheit bilden kann.

In einer besonders bevorzugten Anwendung, die auch als eigenständig erfinderisch angesehen wird, wird die Diamantelektrode und mit dieser insbesondere auch das vorstehend erläuterte Behandlungssystem zur Reinigung eines Dental-Implantat-Teils, insbesondere eines auch als "Pfostenteil" bezeichneten Dentalimplantats, eines auch als "Abutment" bezeichneten Aufbauteils für ein Dentalimplantat, einer Krone oder dergleichen verwendet. Die Behandlung oder Reinigung des Implantat-Teils kann dabei in einer bevorzugten Ausführungsform im inserierten Zustand des Implantats, also insbesondere im Mundraum des Patienten, erfolgen. In einer alternativen, ganz besonders bevorzugten Verwendung kann die Diamantelektrode und mit dieser insbesondere das vorstehend erläuterte Behandlungssystem aber auch außerhalb des Patientenmundes, beispielsweise in einem Behandlungsbad, zur Reinigung und ggf. auch Desinfektion eines Implantat-Teils, insbesondere auch von fabrikneuen Bauteilen, eingesetzt werden.

Von besonderer Bedeutung für das Einwachsverhalten (so genannte Osseointegration) von Implantaten in das Knochengewebe ist nämlich deren Oberfläche im Hinblick auf Hydrophilie: wie sich in Untersuchungen gezeigt hat, begünstigt eine hydrophile Oberfläche nach der Insertion eines Implantats eine gute Durchblutung des umgebenden Knochengewebes, die wiederum für die Ossoeintegration förderlich ist. Daher ist es ein Bestreben bei der Herstellung fabrikneuer Implantate, diese mit einer für die Osseointegration förderlichen hydrophilen Oberfläche bereitzustellen. Es hat sich aber auch gezeigt, dass solchermaßen fabrikseitig vorbereitete Implantate mit der Zeit, insbesondere bei zu langer oder nicht ausreichend sorgfältiger Lagerung, hinsichtlich ihrer Oberflächeneigenschaften degradieren, so dass eine ursprünglich hydrophil ausgestaltete Oberfläche unter Umständen hydrophob werden und damit das Einwachsverhalten des Implantats deutlich verschlechtern kann. Wie sich überraschend herausgestellt hat, kann durch die erfindungsgemäß nunmehr in einer Variante der vorliegenden Erfindung vorgesehene Behandlung eines - ansonsten fabrikneuen - Implantats vor seiner Insertion mit einer Diamantelektrode und dem vorstehend beschriebenen Behandlungskonzept die Hydrophilie der Oberfläche zuverlässig wiederhergestellt werden. Besonders bevorzugt wird daher ein Implantat, insbesondere ein Dentalimplantat, vor seiner Insertion in das Knochengewebe mit dem genannten Behandlungssystem behandelt.

In einer alternativen und ebenfalls als eigenständig erfinderisch abgesehenen Variante wird eine Diamantelektrode der beschriebenen Art, insbesondere das Behandlungssystem der vorstehend genannten Art, zur Desinfektion oder sogar Sterilisation eines Bauteils, beispielsweise eines zur Insertion in die Knochensubstanz vorgesehenen Implantats, oder aber auch von Osteosynthesematerialien wie beispielsweise Platten oder Schrauben oder auch medizinischen oder therapeutischen Instrumenten oder Werkzeugen verwendet. Gerade die im Vorfeld einer Operation oder eines sonstigen Eingriffs in den menschlichen Körper vorzunehmenden Sterilisationsmaßnahmen der medizinischen oder chirurgischen Instrumente können nämlich vergleichsweise aufwändig und mit hohen Vorbereitungszeiten verbunden sein. Wie sich völlig überraschen heraus gestellt hat, kann eine derartige Sterilisation von Instrumenten auf zuverlässige Weise und mit äußerst kurzen Behandlungszeiten in einem Behandlungssystem der genannten Art erfolgen, was deutliche Zeit- und Kostenvorteile mit sich bringt.

Die mit der Erfindung erzielten Vorteile bestehen insbesondere darin, dass durch die Verwendung einer Diamantelektrode in dem Behandlungssystem die für die vorgesehene Prozessführung am behandlungsbedürftigen Bauteil oder Implantat notwendigen Ionen direkt "vor Ort" und in unmittelbarer Nähe der eigentlichen Behandlungszone durch Umwandlung von Wassermolekülen an der Diamantelektrode unter Freisetzung von Hydroxylradikalen und H⁺ - Ionen erzeugt werden können. Diese stellen ein hoch wirksames Reinigungsmittel dar, das für die gezielte Ablösung von Verunreingungen, Biofilm oder Keimen genutzt werden kann.

Ein Ausführungsbeispiel der Erfindung wird anhand einer Zeichnung näher erläutert. Darin zeigen:
- FIG. 1: ein Behandlungssystem zur Reinigung eines mit Biofilm verunreinigten Bauteils,
- FIG. 2: zwei Alternativen eines Reinigungsbads unter Verwendung des Behandlungssystems gem. FIG. 1,
- FIG. 3: ein Behandlungssystem gemäß FIG. 1 im vergrößerten Ausschnitt,
- FIG. 4: eine alternative Ausgestaltung des Behandlungssystems gemäß FIG. 1 im vergrößerten Ausschnitt,
- FIG. 5: ein alternatives Behandlungssystem im ausschnittsweisen Längsschnitt (FIG. 5a), in seitlicher Ansicht (FIG. 5b) und im Querschnitt (FIG. 5c), und
- FIG. 6: ein Sterilisationssystem.

Gleiche Teile sind in allen Figuren mit denselben Bezugszeichen versehen.

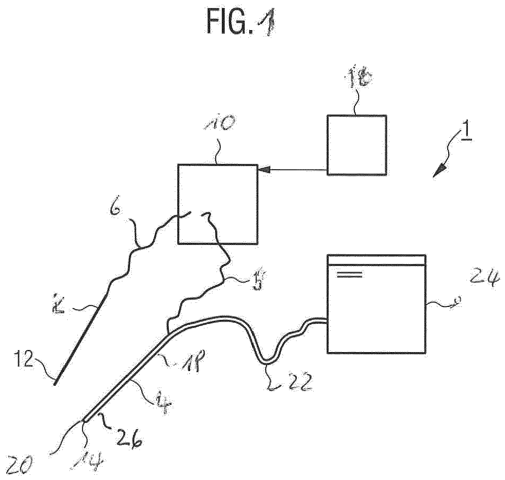

Das Behandlungssystem 1 gemäß in FIG. 1 ist zur Reinigung eines mit Biofilm verunreinigten Bauteils, insbesondere eines Implantat-Teils, und/oder zur Sterilisation eines für den Einsatz im oder am menschlichen Körper vorgesehenen Bauteils oder Instruments vorgesehen. Das Behandlungssystem 1 ist dabei für ein elektrolytisches Reinigungskonzept ausgelegt, bei dem das behandlungsbedürftige Bauteil gezielt und lokalisiert mit Ionen beaufschlagt und dann ein Stromfluss durch das behandlungsbedürftige Bauteil und ggf. die Behandlungsflüssigkeit erzeugt wird. Dazu umfasst das Behandlungssystem 1 zwei Leiterelemente 2, 4, die zur Bildung eines elektrischen Stromkreises jeweils über ein Verbindungskabel 6, 8 an eine elektrische Versorgungseinheit 10 angeschlossen sind.

Das Behandlungssystem 1 ist dabei im Ausführungsbeispiel dafür ausgelegt, den zu Reinigungszwecken des behandlungsbedürftigen Bauteils vorgesehenen Stromfluss gezielt lokalisiert im behandlungsbedürftigen Raumbereich aufbringen zu können. Um dies zu ermöglichen, sind die Leiterelemente 2, 4 in ihrem freien Endbereich in der Art von nadelartig geformten Elektroden ausgebildet, so dass ein Benutzer das jeweilige freie, also nicht über das jeweilige Verbindungskabel 6, 8 mit der Versorgungseinheit 10 verbundene Ende 12 bzw. 14 der nadelartigen Elektrode örtlich sehr genau an einem Zielort positionieren und damit den Strompfad sehr genau einstellen kann. Das Behandlungssystem 1 ist dabei nach dem Auslegungsprinzip aufgebaut, dass der elektrische Strom dem behandlungsbedürftigen Bauteil zugeführt und dieses als Elektrode verwendet werden kann.

Dazu ist im Ausführungsbeispiel gem. FIG. 1 das Leiterelement 2 in der Art einer "konventionellen" Elektrode, also insbesondere als elektrisch leitfähiges nadelartiges Element aus Metall, ausgeführt, kann aber auch aus einem beliebigen anderen leitfähigen Material bestehen. Das Leiterelement 2 ist dabei an seinen Außenseiten mit einer elektrischen Isolierung versehen und weist lediglich an seinem freien Ende 12 eine frei liegende metallische Kontaktspitze auf. Diese kann im Betriebsfall geeignet an das behandlungsbedürftige Bauteil angedrückt und somit ein elektrischer Kontakt zu diesem hergestellt werden. Alternativ kann das Leiterelement 2 natürlich auch an das Bauteil angeklemmt, an dieses angeschraubt oder anderweitig geeignet an diesem befestigt werden. Elektrisch ist das Leiterelement 2 mit einem der Pole der elektrischen Versorgungseinheit 10, insbesondere einer Strom- oder Spannungsquelle, verbunden.

Der elektrischen Versorgungseinheit 10 ist eine Steuereinheit 16 zugeordnet, über die der gelieferte Strom bzw. die gelieferte Spannung steuer- und einstellbar sind.

Des Weiteren umfasst das Behandlungssystem 1 eine Medienkanüle 18, über die eine Behandlungsflüssigkeit geführt und über eine Auslassöffnung 20 ausgebracht werden kann. Die Medienkanüle 18 ist dabei in das zweite Leiterelement 4 integriert und weist somit in ihrem Endbereich ebenfalls eine länglich erstreckte Bauform auf, so dass eine gezielt lokalisierte und kontrollierte Ausbringung der Behandlungsflüssigkeit möglich ist. Medienseitig ist die Medienkanüle 18 über einen Verbindungsschlauch 22 mit einem Vorratsbehälter 24 für die Behandlungsflüssigkeit verbunden. Die Steuereinheit 16 wirkt dabei zusätzlich auch auf ein nicht näher dargestelltes Fördersystem des Verbindungsschlauchs 22, mit dem die Durchflussrate der Behandlungsflüssigkeit durch den Verbindungsschlauch 22 einstellbar ist. Alternativ kann aber selbstverständlich das zweite Leiterelement 4 auch baulich getrennt und unabhängig von der Medienkanüle 18 und dem Verbindungsschlauch 22 ausgeführt sein.

Das Behandlungssystem 1 ist gezielt für eine kombinierte Behandlung des behandlungsbedürftigen Bauteils mit Ionen einerseits und durch Bestromen andererseits ausgelegt. Um dabei aber die Zuführung der für die Behandlung vorgesehenen Ionen von externer Quelle her entbehrlich und das System darüber hinaus auch beim Einsatz in menschlichem Gewebe besonders verträglich zu machen, ist das Leiterelement 4 im Bereich seines freien Endes 14 als Diamantelektrode 26, im Ausführungsbeispiel als bordotierte Diamantelektrode 26, ausgestaltet. Beim Einsatz in wässriger Umgebung und bei Bestromung dieser Diamantelektrode 26 mit geeigneten Parametern, beispielsweise in einer bi-polaren Betriebsweise mit einer Überspannung von wenigen Volt, werden kurzlebige Hydroxylradikale oder OH⁻ - Ionen erzeugt. Bei Kontakt mit organischen Verunreinigungen werden diese direkt zu Kohlendioxid (CO₂) und Wasser (H₂O) oxidiert. Überschüssige Hydroxylradikale stabilisieren sich, indem sie mit Wasser und AD-Soda zu Oxidationsmitteln (Aktivsauerstoff) reagieren. Die dabei gebildeten Percarbonate und Peroxide stellen eine Depotwirkung für die Desinfektion bereit, wodurch die Bildung von unerwünschten Mikroorganismen unterbunden wird bzw. eingetragene Keime deaktiviert werden können.

Gemeinsam mit und korrespondierend zu den Hydroxylradikalen oder OH⁻ -Ionen entstehen infolge der an der Diamantelektrode 26 stattfindenden Zerlegung von Wassermolekülen auch Wasserstoff- oder H⁺ - Ionen, die unmittelbar zur ionischen Behandlung des behandlungsbedürftigen Bauteils genutzt werden können. Bei geeigneter elektrischer Verbindung des zu behandelnden Bauteils über das Leiterelement 2 mit dem Minuspol der elektrischen Versorgungseinheit 10, also bei kathodischer Verschaltung des zu behandelnden Bauteils, wandern die an der Diamantelektrode 26 erzeugten H⁺ - Ionen zum Bauteil hin, wobei sie den an dieses angelagerten Biofilm oder daran angelagerten Verunreinigungen wie Kohlenwasserstoffe penetrieren. An der Oberfläche des behandlungsbedürftigen Bauteils werden die H⁺ - Ionen reduziert und nehmen ein Elektron auf, so dass sich molekularer Wasserstoff (H₂) bildet. Dieser molekulare Wasserstoff entsteht direkt an der Oberfläche des kathodisch geschalteten Bauteils und somit unter dem anhaftenden Biofilm oder den sonstigen anhaftenden Verunreinigungen, wie beispielsweise Kohlenwasserstoffen. Der entstehende Wasserstoff bildet Gasblasen, die anschließend aufsteigen und an die Atmosphäre abgegeben werden. Dabei durchdringen diese Gasblasen den Biofilm bzw. die anderen anhaftenden Verunreinigungen, wobei diese von der Oberfläche des Bauteils abgelöst und "mitgenommen" werden.

Dementsprechend könnte das Behandlungssystem 1 auch ohne die Medienkanüle 18 und somit ohne die Zufuhr einer Behandlungsflüssigkeit genutzt werden, falls sich - beispielsweise im Mundraum des Patienten bei der Behandlung eines Dentalimplantats - im Umfeld des Behandlungsvorgangs ausreichend Wasser zur Bildung der genannten Radikale befindet. Die Medienkanüle ist aber bevorzugt vorgesehen, um in jedem Fall eine ausreichende Versorgung mit Wasser als Grundstoff für die Ionenproduktion sicherzustellen und insbesondere in der Art einer Spülung den Abtransport und die stetige Verdünnung der abgelösten Verunreinigungen zu gewährleisten. Dementsprechend ist bevorzugt als Behandlungsflüssigkeit auch eine Flüssigkeit auf Wasserbasis oder mit ausreichend hohem Wasseranteil vorgesehen.

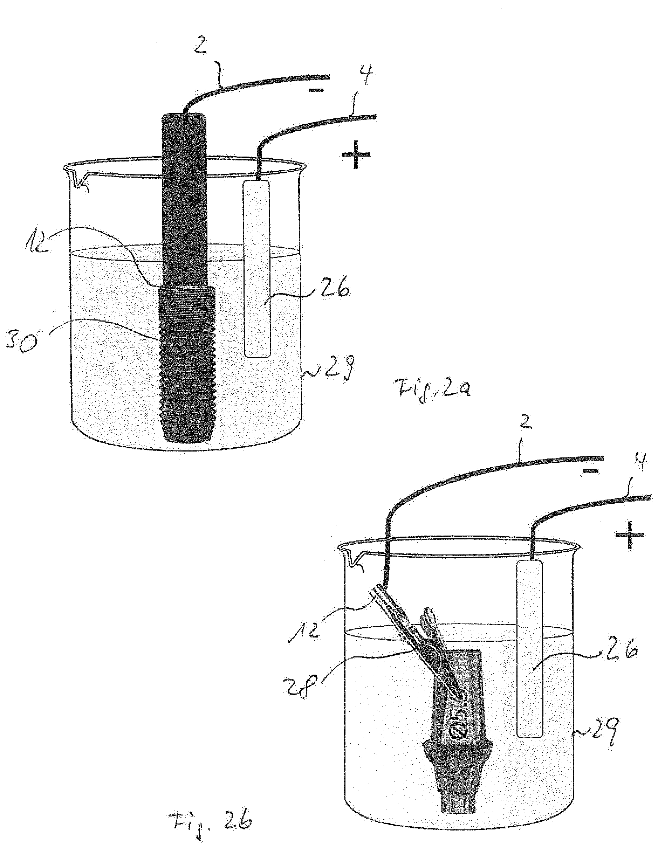

Ein Ausführungsbeispiel für diese Variante, bei der die Medienkanüle 18 nicht unbedingt notwendig ist, ist in FIG. 2 für zwei Einsatzarten, nämlich für die Aufbereitung und/oder Sterilisation eines fabrikneuen Dentalimplantats 30 (FIG. 2a) und für die Aufbereitung und/oder Sterilisation eines Abutments für ein Dentalimplantat 30 (Fig. 2b), gezeigt. Diese in FIG. 2 dargestellte, als eigenständig erfinderisch angesehene Verwendung der Diamantelektrode 26 zu Reinigungs- oder Sterilisationszwecken ist für eine Vielzahl von Bauteilen geeignet und bevorzugt, beispielsweise für Implantate jeglicher Art und ihre Bau- oder Bestandteile, für chirurgische oder sonstige medizinische Instrumente, insbesondere unmittelbar vor ihrem Einsatz im oder am menschlichen Körper, für Gingivaformer und dergleichen.

In der in FIG. 2 dargestellten Variante ist der Einsatz des Behandlungssystems 1 in der Art eines Wasserbades vorgesehen. Das behandlungsbedürftige Bauteil, beispielsweise das Dentalimplantat 30, wird dabei mit dem freien Ende 12 des Leiterelements 2 verbunden. Dies kann, wie beispielhaft in FIG. 2b gezeigt, über eine an das Leiterelement 2 endseitig angeschlossene Klemme 28 erfolgen. Wie in FIG. 2 gezeigt, wird das jeweilige Bauteil, beispielsweise das Dentalimplantat 30, zur Behandlung in ein in einem Behälter 29 vorgehaltenes Reinigungsbad eingetaucht. Das Reinigungsbad enthält dabei eine geeignet gewählte Behandlungsflüssigkeit auf Wasserbasis, so dass die oben beschrieben Erzeugung der Hydroxylradikale und H⁺ - Ionen stattfinden kann. Weiterhin wird die Diamantelektrode 26 in das Reinigungsbad eingetaucht. Zur Behandlung wird dann über die Versorgungseinheit 10 ein Stromfluss durch das Bauteil, das Reinigungsbad und die Diamantelektrode 26 eingestellt, so dass in der vorstehend beschriebenen Weise die zur Reinigung und Sterilisation vorgesehenen Radikale erzeugt werden und das Bauteil dekontaminieren. Besonders vorteilhaft wird eine derartige Behandlung an einem fabrikneuen, zur Insertion vorgesehenen Dentalimplantat 30 vorgenommen, um auf diese Weise zuverlässig die Hydrophilie der entsprechend fabrikseitig vorbereiteten Oberfläche wiederherzustellen.

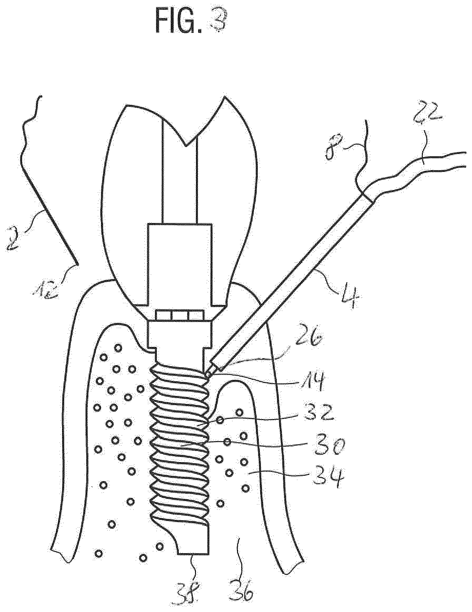

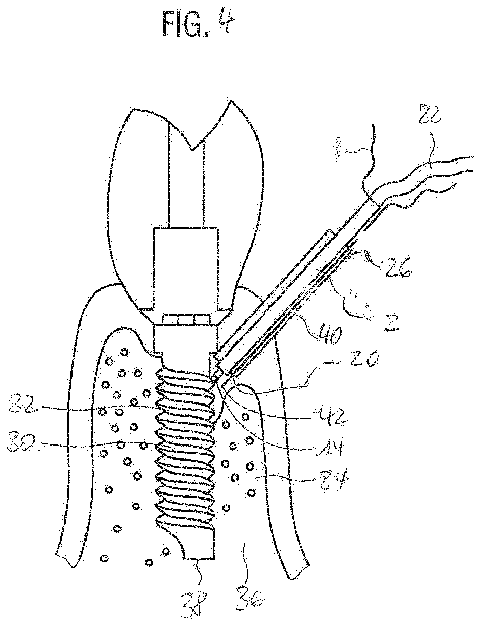

Alternativ kann das Behandlungssystem 1 aber auch zur Behandlung eines in den Kieferknochen eines Patienten bereits inserierten Dentalimplantats 30 verwendet werden, wie dies in den Ausführungsbeispielen gem. FIG. 3 und FIG. 4 gezeigt ist.

In diesen Ausführungsbeispielen ist weiterhin die Nutzung der elektrischen Leitfähigkeit der in der Medienkanüle 18 geführten Behandlungsflüssigkeit zur Bildung einer der Elektroden des Stromkreises vorgesehen. Dabei ist die Medienkanüle 18 in das als Diamantelektrode 26 ausgestaltete Leiterelement 4 integriert, so dass eine besonders kompakte Bauweise erreichbar ist. Dabei ist der Innenraum der Medienkanüle 18 seinerseits an das Verbindungskabel 8 angeschlossen und über dieses elektrisch mit einem der Pole der elektrischen Versorgungseinheit 10 verbunden. Damit bildet die Auslassöffnung 20 der Medienkanüle 18 in elektrischer Hinsicht einen Kontakt oder elektrischen Kontaktpunkt, über den der Stromfluss in das behandlungsbedürftige Bauteil erfolgt. Bei geeigneter Positionierung der Medienkanüle 18 und ihrer Auslassöffnung 20 möglichst in unmittelbarer Nähe zum behandlungsbedürftigen Bauteil und durch die Nutzung der Auslassöffnung 20 als elektrischen Kontakt ist erreicht, dass der zu Behandlungs- und Reinigungszwecken angelegte elektrische Strom durch die von den Bakterien befallene Oberflächenzone des behandlungsbedürftigen Bauteils hindurch- und von dort aus weitgehend unmittelbar, also insbesondere ohne "Umwege" über weiteres Körpergewebe oder dergleichen, zur als Kontaktfläche dienenden Auslassöffnung 20 fließen kann. Die Medienkanüle 18 einschließlich der darin geführten, elektrisch leitfähigen Behandlungsflüssigkeit und den entsprechenden Anschlusselementen bildet somit im Ausführungsbeispiel das zweite, einen elektrischen Strompfad zur Auslassöffnung 20 bildende Leiterelement.

Wie der vergrößerten Darstellung gemäß FIG. 3 entnehmbar ist, ist das im Bereich seines freien Endes 14 als Diamantelektrode 26 ausgestaltete Leiterelement 4 zur bedarfsweisen lokalisierten Positionierung in unmittelbarer Nähe zum behandlungsbedürftigen Bauteil ausgelegt, so dass die bei der Behandlung vorgesehene Produktion der Hydroxylradikale und H⁺ - Ionen in unmittelbarer Nähe zum Verwendungsort erfolgen kann. Im Ausführungsbeispiel wird dies anhand eines in den Mundknochen eines Patienten inserierten Dentalimplantats 30 erläutert; selbstverständlich sind aber auch andere Anwendungen denkbar, bei denen auf flexible und fokussierte Weise ein Bauteil, beispielsweise ein Knochenimplantat beliebiger Bauweise, vom Befall eines Biofilms gereinigt werden soll. In FIG. 3 ebenfalls dargestellt ist ein dem Dentalimplantat 30 im Bereich seines Außengewindes 32 benachbarter, räumlich begrenzter Raumbereich 34 im Kieferknochen 36, der von Periimplantitis befallen und dementsprechend mit Bakterien belastet ist.

Generell besteht bei Dental-Implantatsystemen, insbesondere auch bei zweiteiligen Implantatsystemen, das Problem, dass durch ein Eindringen von Bakterien oder Keimen in den Gewebebereich in der Nähe der Insertionsstelle, insbesondere im Bereich des in den Kiefer eingebrachten Außengewindes 32, Entzündungen oder Entzündungsherde entstehen können. Derartige, insbesondere auch in Folge einer so genannten Periimplantitis entstehende Entzündungen können, insbesondere wenn sie sich über einen längeren Zeitraum entwickeln und verfestigen können, zu einer gravierenden Beeinträchtigung des Gewebes und des Knochens im Bereich der Insertionsstelle führen. Ohne geeignete Gegenmaßnahmen können diese Beeinträchtigungen dazu führen, dass das gesamte Implantatsystem wieder aus dem Knochen entfernt und durch andere Prothetik ersetzt werden muss. Dieser durch die Periimplantitis hervorgerufene, äußerst unerwünschte Effekt kann somit zu einem Totalverlust des Implantatsystems führen, so dass erneute chirurgische Maßnahmen wie beispielsweise ein Ausschaben des betroffenen Bereichs im Kieferknochen und die Neuversorgung mit einem Implantatsystem notwendig werden können. Durch eine derartige Entnahme kann es zudem zu Knochenverlust oder sonstigem Verlust an Gewebesubstanz kommen, die im Extremfall soweit führen kann, dass eine Neuversorgung mit einem anderen Implantat gar nicht mehr möglich ist. Eine derartige durch Periimplantitis hervorgerufene Notwendigkeit einer Neuversorgung kann auch nach vergleichsweise langen Zeiträumen nach dem ersten Einsetzen des Implantatsystems von beispielsweise bis zu einigen Jahren oder sogar Jahrzehnten auftreten.

Die im Zusammenhang mit einer Periimplantitis beobachteten Keime oder Bakterien können dabei grundsätzlich das Innere der Komponenten des Dentalimplantats 30 besiedeln, haften aber in der Regel bevorzugt direkt an der Oberfläche des in den Kieferknochen 36 inserierten Dentalimplantats 30 im Kontaktbereich mit dem umgebenden Gewebe oder Knochenmaterial, also insbesondere im Bereich des Außengewindes 32, an. In dessen Bereich kann die Oberfläche des Dentalimplantats 30 mit einer Aufrauhung oder dergleichen versehen sein, um das Einwachsen in das Gewebe oder den Knochen besonders zu begünstigen und das Einheilen des Dentalimplantats 30 nach der Insertion zu unterstützen. Gerade im Bereich einer derartigen, eigentlich als besonders günstig für das Implantatsystem angesehenen Aufrauhung der Oberfläche kann aber die Ansiedlung der Keime oder Bakterien vermehrt stattfinden, wobei die Rauigkeit ein gezieltes Entfernen der vorhandenen Keime oder Bakterien noch zusätzlich erschwert.

Es besteht daher der dringende Wunsch nach geeigneten Gegenmaßnahmen, um für den Fall einer sich anbahnenden oder bereits aufgetretenen Periimplantitis oder Mukositis unter Erhaltung des bereits eingesetzten Implantatsystems wirksam den Entzündungsherd bekämpfen und die eingedrungenen Keime abtöten zu können, so dass sich anschließend wieder gesundes Gewebe oder gesunde Knochensubstanz im Bereich um das Außengewinde 32 herum bilden kann. Dazu ist wünschenswert, zusätzlich zu einem gezielten Abtöten der Keime oder Bakterien im betroffenen Bereich auch noch deren Materialreste und Fragmente, ggf. ebenso wie extrazelluläre Matrix und Enotoxine, zuverlässig aus dem betroffenen Raumbereich zu entfernen, so dass anschließend der betroffene Bereich wieder von gesundem Gewebe oder Knochenmaterial ausgefüllt werden und sich wieder eine innige Verbindung zwischen der Außenoberfläche des Dentalimplantats 30 und dem umgebenden Gewebe oder Knochenmaterial bilden kann. Zudem sollte der von der Bakterienbeschichtung gebildete Biofilm einschließlich der organischen Reste abgetöteter Bakterien zuverlässig entfernt werden.

Zu diesem Zweck, also zum Abtöten von Keimen oder Bakterien im Insertionsbereich des Dentalimplantats 30 und insbesondere auch zum anschließenden Ausspülen, Entfernen und Austragen der Gewebe- und Materialreste der abgetöteten Bakterien, ist das Behandlungssystem 1 vorgesehen. Dieses beruht hinsichtlich seiner Ausgestaltung und prinzipiellen Ausführung auf zwei jeweils als eigenständig erfinderisch angesehenen Grundkonzepten: Einerseits ist es dafür ausgestaltet, die im Insertionsbereich des Dentalimplantats 30 vorhandenen Keime oder Bakterien durch lokale Erzeugung und anschließende gezielte Zuführung eines bakterioziden, aber für den menschlichen Organismus verträglichen Reinigungs- oder Desinfektionsmittels gezielt abzutöten. Andererseits ist es dafür ausgelegt, eventuell an der Oberfläche des Dentalimplantats 30, insbesondere im Bereich Außengewindes 32, noch anhaftende Reste oder Fragmente von Keimen und/oder Bakterien durch eine geeignete Beaufschlagung mit Strom oder Stromstößen von der Außenoberfläche des Dentalimplantats 30 abzulösen, so dass sie anschließend ausgewaschen werden können.

In einem sowohl bezüglich der Ausgestaltung des Systems als auch bezüglich der vorgesehenen Verfahrensschritte des Behandlungsverfahrens jeweils eigenständig als erfinderisch angesehenen Aspekt ist das Behandlungssystem 1 daher sowohl strukturell als auch funktional/konzeptionell dafür ausgelegt, die zum Abtöten der Keime oder Bakterien und/oder zum Reinigen des inserierten Implantat-Teils vorgesehenen Ionen bzw. Radikale gezielt und erst während der eigentlichen Prozessführung in unmittelbarer Nähe des Insertionsbereichs des Dentalimplantats 30, insbesondere des Bereichs von dessen Außengewinde 32, zu erzeugen. Dies erfolgt durch den Betrieb der als Diamantelektrode 26 ausgelegten Endbereichszone des Leiterelements 4.

Für die Ablösung der Bakterien oder Keime bzw. deren Resten oder Fragmenten von der Oberfläche ist deren Behandlung durch Beaufschlagung mit einem Gleichstrom oder alternativ oder zusätzlich mit gepulsten Stromstößen vorgesehen. Wie sich nämlich ebenfalls völlig überraschend herausgestellt hat, scheint gerade diese gepulste Beaufschlagung mit Stromstößen in Kombination mit geeignet gewählten lonenkonzentrationen in der Behandlungsflüssigkeit besonders zuverlässig das Ablösen der Bakterien oder Keime bzw. von deren Fragmenten oder Resten von der darunterliegenden Oberfläche zu bewirken, selbst wenn diese aufgeraut ist und eigentlich das Anhaften organischen Materials auf Grund ihrer Oberflächenstruktur besonders begünstigt.

In der in FIG. 3 dargestellten Ausführungsform sind das erste Leiterelement 2 einerseits und das zweite Leiterelement 4 mit der in dieses integrierten Medienkanüle 18 andererseits als voneinander im Wesentlichen unabhängige Bauteile ausgeführt, die elektrisch mit der gemeinsamen Versorgungseinheit 10 verbunden sind. Das Leiterelement 2 ist dabei im in FIG. 3 gezeigten Ausführungsbeispiel in der Art einer "konventionellen" Elektrode, also insbesondere als elektrisch leitfähiges nadelartiges Element aus Metall, ausgeführt. Durch diese Ausführung können die Leiterelemente 2,4 unabhängig voneinander bewegt und positioniert werden, so dass eine besonders flexible Bearbeitung des behandlungsbedürftigen Bauteils ermöglicht ist. Insbesondere können dabei die zur Kontaktierung jeweils vorgesehenen freien Enden 12, 14 unabhängig voneinander und damit gegebenenfalls für einen optimierten elektrischen Kontakt zum Bauteil geeignet an diesem positioniert werden.

Demgegenüber ist im Ausführungsbeispiel gemäß FIG. 4 eine Variante gezeigt, bei der die Diamantelektrode 26, das Leiterelement 2 und ggf. auch noch die Wasserversorgung, d. h. die Medienkanüle 18, in ein gemeinsames Gehäuse 40 integriert sind. Im gezeigten Ausführungsbeispiel sind der elektrische Leiter des Leiterelements 2 und die Diamantelektrode 26 koaxial zueinander geführt, wobei die Diamantelektrode 26 das Leiterelement konzentrisch umgibt. Die Kontaktspitze des Leiterelements 2 kann dabei zur Herstellung des kathodischen elektrischen Kontakts bis zum Dentalimplantat 30 herangeführt und mit diesem in Kontakt gebracht werden. Die Auslassöffnung 20 der Medienkanüle 18 und somit der Wasserversorgung kann entsprechend benachbart dazu positioniert werden. Im Allgemeinen ist durch eine derartige integrierte Bauweise mit dem für Medienkanüle 18 und Leiterelement 2 gemeinsamen Gehäuse 40 eine erleichterte Handhabung erreichbar, da für den Behandler damit auch eine einhändige Bedienung und Positionierung des Systems möglich ist.

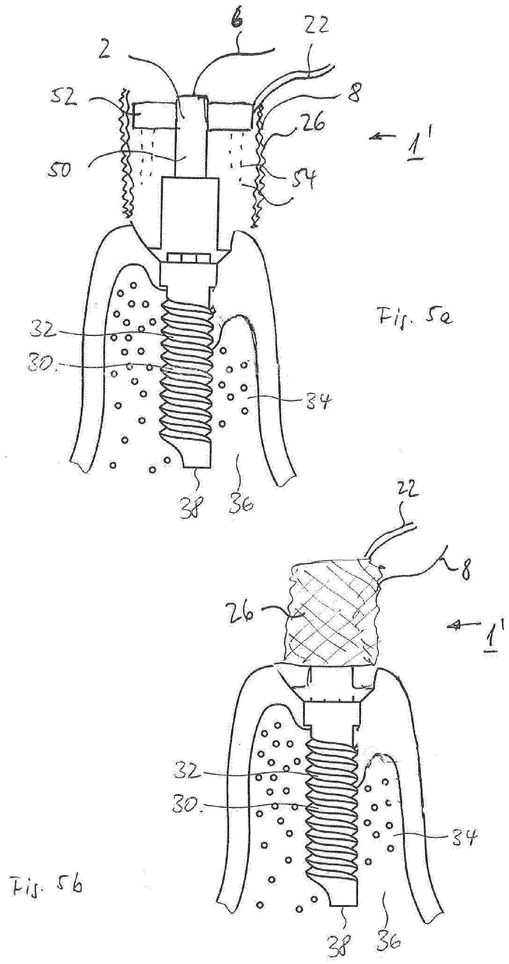

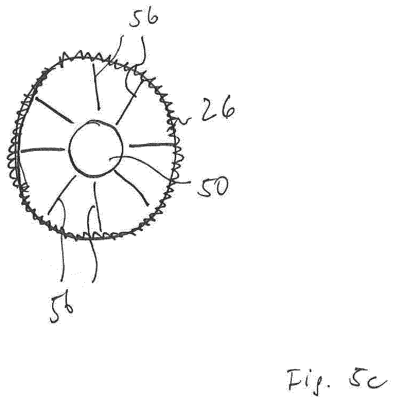

Ein alternatives, in seiner Ausgestaltung als eigenständig erfinderisch angesehenes Behandlungssystem 1' ist in ausschnittsweiser Vergrößerung in FIG. 5 sowohl im Längsschnitt (FIG. 5a), in seitlicher Ansicht (FIG. 5b) und im Querschnitt (FIG. 5c) gezeigt. Dieses auch als "Duschkopfsystem" bezeichnete Behandlungssystem 1' umfasst als erstes Leiterelement 2 einen fest mit dem Dentalimplantat 30 verbundenen Kontaktkörper 50, der über das Verbindungskabel 6 mit dem Minuspol der in FIG. 5 nicht näher dargestellten elektrischen Versorgungseinheit 10 verbunden ist. Der Kontaktkörper 50 kann dabei beispielsweise über ein im Implantat 30 vorhandenes Schraubgewinde oder auch auf andere geeignete Weise mit dem Dentalimplantat 30 verbunden sein. Durch die elektrische Kontaktierung ist das Dentalimplantat 30 somit kathodisch geschaltet.

In einem oberen Endbereich des Kontaktkörpers 50 ist ein Verteilerelement 52 angeordnet, das über den Verbindungsschlauch 22 mit dem Reservoir für eine Behandlungsflüssigkeit, im Ausführungsbeispiel Wasser, verbunden ist. Das Verteilerelement 52 bildet eine Art Ringraum um das obere Ende des Kontaktkörpers 50, der vom zugeführten Wasser durchströmt werden kann. Nach unten hin ist dieser Ringraum des Verteilerelements 52 perforiert ausgeführt, so dass das darin geführte Wasser nach unten hin (vergleichbar der Wirkungsweise eines Duschkopfs) tröpfchenweise austreten kann. Dies ist in FIG. 5a durch die dargestellten Tröpfchen 54 angedeutet.

In der Art einer konzentrischen Anordnung um das Kontaktelement 50 und in dessen oberem Bereich um das Verteilerelement 54 herum ist die Diamantelektrode 26 angeordnet, die in dieser Ausführungsform als Hohlzylinder ausgestaltet ist. Zur Bereitstellung einer besonders großen wirksamen Oberfläche ist die Diamantelektrode 26 dabei mit einer strukturierten Oberfläche, beispielsweise als Gitter- oder Wabenstruktur, ausgeführt. Wie in den anderen Ausführungsformen auch kann die Diamantelektrode dabei insbesondere auf der Basis eines metallischen, keramischen oder anderweitig geeignet gewählten Grundkörpers ausgeführt sein, der an seiner Oberfläche mit einer geeigneten, vorzugsweise bordotierten, Diamantbeschichtung versehen ist. Die Diamantelektrode 26 ist über das Verbindungskabel 8 an den Pluspol der elektrischen Versorgungseinheit 10 angeschlossen und somit anodisch geschaltet.

Zur weiteren Verdeutlichung ist das Behandlungssystem 1' in FIG. 5b mit seitlicher Ansicht der Diamantelektrode 26 gezeigt. Dabei ist die strukturierte Oberfläche der Diamantelektrode 26 deutlich erkennbar.

Der Querschnittsdarstellung in FIG. 5c ist entnehmbar, dass das Kontaktelement 50 Konzentrisch von der Diamantelektrode 26 umgeben ist. Dazwischen ist eine Anzahl von Abstandshaltern 56 aus geeignet gewähltem Isolatormaterial angeordnet. Des Weiteren kann die Diamantelektrode 26 außenseitig von einem geeigneten Isolatormantel umgeben sein.

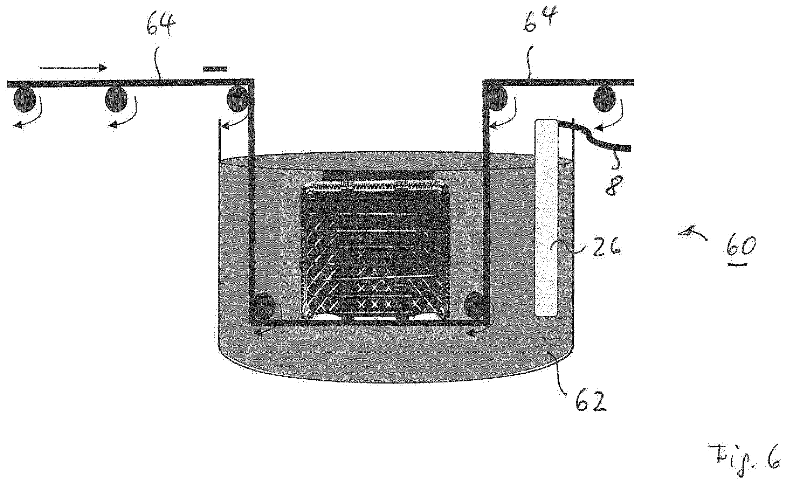

Eine ganz besonders bevorzugte, ebenfalls als eigenständig erfinderisch angesehene Verwendung einer Diamantelektrode 26 zur Desinfektion oder Sterilisation eines Bauteils, beispielsweise eines zur Insertion in die Knochensubstanz vorgesehenen Implantats, oder aber auch von Osteosynthesematerialien wie beispielsweise Platten oder Schrauben oder auch medizinischen oder therapeutischen Instrumenten oder Werkzeugen, ist in FIG. 6 beispielhaft für ein Sterilisationssystem 60 gezeigt. Das Sterilisationssystem 60 umfasst ein Behandlungsbad 62, in dem Wasser oder eine wässrige Flüssigkeit als Behandlungsflüssigkeit vorgehalten ist. In das Behandlungsbad 62 ist die Diamantelektrode 26 eingetaucht. Die Diamantelektrode 26 ist analog zu den vorstehend beschriebenen Varianten über das Verbindungskabel 8 an den Pluspol der elektrischen Versorgungseinheit 10 angeschlossen und somit anodisch geschaltet.

Durch das Behandlungsbad 62 hindurch ist ein Förderband 64 geführt, das geeignet mit dem Minuspol der elektrischen Versorgungseinheit verbunden und somit kathodisch geschaltet ist. Auf dem Förderband 64 werden die zu sterilisierenden Bauteile oder Instrumente transportiert und durch das Behandlungsbad 64 hindurch befördert. Während des Durchlaufs erfolgt durch geeignete Bestromung unter Nutzung des vorstehend beschriebenen Prozesses und der dabei stattfindenden Erzeugung der H⁺ - Ionen die Ablösung von sich auf den Instrumenten oder Bauteilen eventuell befindlichem Biofilm oder anderen Verunreinigungen, so dass eine Sterilisation erreicht werden kann.

Der besondere Vorteil einer derartigen Ausgestaltung des Sterilisationssystems 60 besteht insbesondere darin, dass eine zuverlässige Desinfektion oder Sterilisation im Durchlaufbetrieb und damit einhergehend mit vergleichsweise großer Durchsatz- und Behandlungsgeschwindigkeit erfolgen kann. Im Vergleich zu herkömmlichen Sterilisationsverfahren beispielsweise für medizinische oder zahnmedizinische Instrumente können damit deutlich kürzere Behandlungszeiten erreicht werden, die mit entsprechend erheblichen Kosten- und Logistikvorteilen einhergehen.

### Bezugszeichenliste

- 1: Behandlungssystem
- 2,4: Leiterelement
- 6, 8: Verbindungskabel
- 10: Versorgungseinheit
- 12, 14: Ende
- 16: Steuereinheit
- 18: Medienkanüle
- 20: Auslassöffnung
- 22: Verbindungsschlauch
- 24: Vorratsbehälter
- 26: Diamantelektrode
- 28: Klemme
- 29: Behälter
- 30: Dentalimplantat
- 32: Außengewinde
- 34: Raumbereich
- 36: Kieferknochen
- 38: Pfostenteil
- 40: Gehäuse
- 42: Ende
- 50: Kontaktkörper
- 52: Verteilerelement
- 54: Tröpfchen
- 56: Abstandshalter
- 60: Steril isationssystem
- 62: Behandlungsbad
- 64: Förderband

## Patentansprüche

1. Behandlungssystem (1) zur Reinigung eines mit Biofilm verunreinigten Bauteils, insbesondere zur Reinigung von bakteriell verunreinigten Oberflächen von Knochenimplantaten oder Dentalimplantaten (30), mit zumindest zwei zur Bildung eines elektrischen Stromkreises an eine elektrische Versorgungseinheit (10) angeschlossenen Leiterelementen (2,4), von denen eines mit dem behandlungsbedürftigen Bauteil in elektrischen Kontakt bringbar ist, **dadurch gekennzeichnet, dass** zumindest eines der Leiterelemente (2,4) als bordotierte Diamantelektrode (26) ausgeführt ist.

2. Behandlungssystem (1) nach Anspruch 1, das eine zur Ausbringung einer Behandlungsflüssigkeit vorgesehene Medienkanüle (18) aufweist.

3. Behandlungssystem (1) nach Anspruch 2, bei dem das Leiterelement (2,4) und die Medienkanüle (18) in einem gemeinsamen Gehäuse (40) angeordnet sind.

4. Behandlungssystem (1) nach Anspruch 3, bei dem der Innenraum der Medienkanüle (18) elektrisch leitend mit einem Pol der elektrischen Versorgungseinheit (10) verbunden ist.

## Claims

1. A treatment system (1) for cleaning a component with biofilm impurities, in particular for cleaning bacterially contaminated surfaces of bone implants or dental implants (30), having at least two conductor elements (2, 4) connected to an electrical supply unit (10) for forming an electrical circuit, one of which can be brought into electrical contact with the component in need of treatment, **characterized in that** at least one of the conductor elements (2, 4) is designed as a boron-doped diamond electrode (26).

2. The treatment system (1) of claim 1, including a media cannula (18) provided for yielding a treatment fluid.

3. The treatment system (1) of claim 2, wherein the conductor element (2, 4) and the media cannula (18) are arranged in a common housing (40).

4. The treatment system (1) of claim 3, wherein the inner space of the media cannula (18) is connected with a pole of the electrical supply unit (10) in an electrically conductive manner.

## Revendications

1. Système de traitement (1) destiné à nettoyer un composant souillé par biofilm, en particulier destiné à nettoyer des surfaces souillées bactériellement, d'implants osseux ou d'implants dentaires (30), ayant au moins deux éléments conducteurs (2, 4) reliés à une unité d'alimentation électrique (10) pour former un circuit électrique, l'un des deux éléments conducteurs pouvant être mis en contact électrique avec le composant ayant besoin de traitement, **caractérisé en ce que** au moins un des éléments conducteurs (2, 4) est conçu comme une électrode diamant (26) dopée de bore.

2. Système de traitement (1) selon la revendication 1, comprenant une canule à milieu (18) pourvue pour délivrer un fluide de traitement.

3. Système de traitement (1) selon la revendication 2, dans lequel l'élément conducteur (2, 4) et la canule à milieu (18) sont disposés dans un boîtier (40) commun.

4. Système de traitement (1) selon la revendication 3, dans lequel l'espace intérieur de la canule à milieu (18) est relié de manière électriquement conductive à un pôle de l'unité d'alimentation électrique (10).
